# EUROPÄISCHE PATENTSCHRIFT

(11) **EP 3 908 566 B1**
(45) Veröffentlichungstag und Bekanntmachung des Hinweises auf die Patenterteilung: **26.07.2023**
(21) Anmeldenummer: 20700208.0
(22) Anmeldetag: 10.01.2020
(51) Int. Cl.: C07C 29/60, C07C 29/10, C07C 33/20

(54) **HERSTELLUNG VON 5-ARYL-PENTANOLEN**
PREPARATION OF 5-ARYL PENTANOLS
PRODUCTION DE 5-ARYLE-PENTANOLS

(30) Priorität: 11.01.2019 EP 19151386
(43) Veröffentlichungstag der Anmeldung: 17.11.2021
(73) Patentinhaber: BASF SE, 67056 Ludwigshafen am Rhein (DE)
(72) Erfinder: STOCK, Christoph, 67056 Ludwigshafen (DE); DE WISPELAERE, Irene, 2040 Antwerpen (BE); BRUNNER, Bernhard, 67056 Ludwigshafen (DE); KRAUSE, Wolfgang, 68623 Lampertheim (DE); PELZER, Ralf, 68623 Lampertheim (DE)
(74) Vertreter: BASF IP Association
(86) Internationale Anmeldenummer: PCT/EP2020/050490
(87) Internationale Veröffentlichungsnummer: WO 2020/144307

(56) Entgegenhaltungen:
- EP-A2- 1 298 118
- CH-A5- 655 932
- YADAV ET AL: "Enzymatic kinetic resolution of racemic 4-tetrahydropyranols by Candida rugosa lipase", TETRAHEDRON LETTERS, ELSEVIER, AMSTERDAM, NL, Bd. 48, Nr. 26, 25. Juni 2007 (2007-06-25) , Seiten 4631-4633, XP022097631, ISSN: 0040-4039, DOI: 10.1016/J.TETLET.2007.04.091

## Beschreibung

### HINTERGRUND DER ERFINDUNG

Die vorliegende Erfindung betrifft ein Verfahren zur Herstellung von 5-Aryl-pentanolen aus 2-Aryl-4-hydroxy-tetrahydropyranen als Ausgangsstoffen.

### STAND DER TECHNIK

Für den steigenden Bedarf an Riechstoffen mit interessanten Duftnoten braucht es nicht zwangsläufig neue olfaktorisch aktive Moleküle. Auch eine neue Kombination von bekannten Aroma- und Geschmacksstoffen kann zu neuen Geruchsprofilen führen. Dringend gesucht sind deshalb neue und einfache Synthesemöglichkeiten bekannter Aroma- und Geschmacksstoffe.

5-Aryl-pentanole haben weite Verbreitung als Aroma- und Geschmacksstoffe gefunden. Ein bekannter Vertreter aus dieser Klasse ist das 3-Methyl-5-phenyl-pentan-1-ol (Phenoxanol ^{®}), welches eine florale Duftnote aufweist.

Die CH 655 932 beschreibt ein Verfahren zur Herstellung von 5-Aryl-3-methyl-pentanol durch Hydrogenolyse von Pyranderivaten der Formel 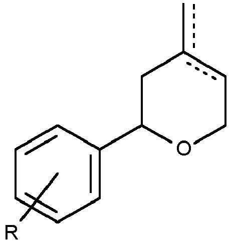 und speziell von 2-Phenyl-4-methylenpyran in Gegenwart eines Metallkatalysators und einer sauren Substanz wie z. B. einer Protonensäure oder einer sauren Diatomeenerde.

US 2003/0060667 beschreibt ein Verfahren zur Herstellung von 3-Methyl-5-phenyl-pentanol durch Hydrogenolyse von 6-Phenyl-4-methyl-5,6-dihydro-2H-pyran in Gegenwart eines geträgerten Palladium-Katalysators ohne zusätzliche Verwendung einer sauren Substanz.

U. Matteoli et al., Tetrahedron Asymmetry 2007, 18, 797 beschreibt ein enatioselektives Verfahren zur Herstellung von Phenoxanol^{®} (3-Methyl-5-phenylpentanol), Citralis^{®} (3-Methyl-5-phenylpentanal) und Citralis Nitrile^{®} (3-Methyl-5-phenylpentane-1-nitril). Das Verfahren basiert auf einer asymmetrischen Hydrierung von (Z)- oder (E)-3-Methyl-5-phenyl-pent-2-en-1-ol in Gegenwart eines Iridiumkatalysators. Zunächst wird Phenoxanol erhalten, welches zu Citralis oxidiert wird. Citralis kann in einer weiteren Reaktion zu Citralis Nitrile umgesetzt werden.

S. Superchi et al., Chirality 2011, 23, 761-767 beschreibt eine Kupfer-katalysierte asymmetrische Additionsreaktion von Dimethylzink an Diethyl-3-phenylpropyliden-malonat zur Herstellung von Phenoxanol^{®}. Diese Reaktion findet in Gegenwart von chiralen Phosphitliganden statt. Das erhaltene Phenoxanol^{®} kann in einer anschließenden Reaktion zu Citralis^{®} und Citralis Nitrile^{®} umgesetzt werden.

P.G. Anderson et al., ACS Catalysis 2016, 6, 8342 beschreibt die Herstellung von γ,γ-disubstituierten und β,γ-disubstituierten Allylalkoholen. Die Allylalkohole werden in Gegenwart eines Iridium-basierten N,P-Katalysators hydriert, wobei die entsprechenden Hydrierprodukte erhalten werden.

Es besteht weiterhin ein großer Bedarf an effektiven Verfahren zur Herstellung von 5-Aryl-pentanolen aus leicht verfügbaren Ausgangsstoffen.

Der vorliegenden Erfindung liegt die Aufgabe zugrunde, ein verbessertes Verfahren zur Herstellung von 5-Aryl-pentanolen zur Verfügung zu stellen.

Überraschenderweise wurde jetzt gefunden, dass durch Hydrierung von 2-Aryl-4-hydroxy-tetrahydropyranen in Gegenwart eines Hydrierkatalysators unter sauren Bedingungen auf schnellem Weg 5-Aryl-pentanole, speziell 3-Methyl-5-phenyl-pentan-1-ol, erhalten werden können.

### ZUSAMMENFASSUNG DER ERFINDUNG

Gegenstand der Erfindung ist ein Verfahren zur Herstellung von Verbindungen der allgemeinen Formel (I) 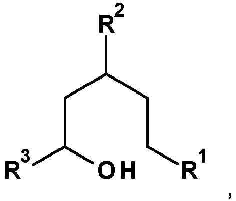 wobei
- R¹: ausgewählt ist unter
unsubstituiertem oder mit 1, 2, 3, 4 oder 5 Substituenten, ausgewählt unter C₁-C₁₂-Alkyl, C₁-C₁₂-Alkoxy, Phenyl und Benzyl, substituiertem Aryl mit 6 bis 20 Kohlenstoffatomen;
- R²: ausgewählt ist unter Wasserstoff und C₁-C₆-Alkyl;
- R³: ausgewählt ist unter Wasserstoff und C₁-C₆-Alkyl;
umfassend die Schritte:
a) Bereitstellung wenigstens einer Verbindung der allgemeinen Formel (II) 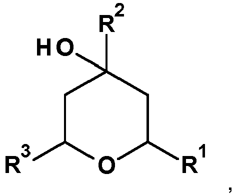 wobei R¹, R² und R³ die zuvor angegebenen Bedeutungen haben,
b) Hydrierung der Verbindung (II) in Gegenwart eines Hydrierkatalysators unter sauren Bedingungen.

### BESCHREIBUNG DER ERFINDUNG

Das erfindungsgemäße Verfahren weist die folgenden Vorteile auf:
- Mit der erfindungsgemäß vorgesehenen Reaktion wird ein Zugang zu 5-Aryl-pentanolen und speziell zu 3-Methyl-5-phenyl-pentan-1-ol (Phenoxanol^{®}) ermöglicht, der nur eine Reaktionsstufe erfordert (Eintopfsynthese).
- Das hergestellte 3-Methyl-5-phenyl-pentan-1-ol (Phenoxanol^{®}) kann als Ausgangstoff zur Herstellung von Citralis^{®} und Citralis Nitrile^{®} verwendet werden.
- Zur Herstellung der 5-Aryl-pentanole und speziell von 3-Methyl-5-phenyl-pentan-1-ol müssen keine weiteren teuren und/oder potentiell gefährlichen Reagenzien eingesetzt werden.

Sofern im Folgenden nicht genauer angegeben, umfasst die Erfindung alle möglichen Isomeren, d. h. alle mögliche Entatiomere und Diastereomere, alle Enantiomere in Reinform sowie racemische und optisch aktive Gemische der Enantiomeren dieser Verbindungen.

Im Rahmen der vorliegenden Erfindung steht der Ausdruck geradkettiges oder verzweigtes Alkyl vorzugsweise für C₁-C₆-Alkyl und besonders bevorzugt für C₁-C₄-Alkyl. Alkyl steht insbesondere für Methyl, Ethyl, n-Propyl, Isopropyl, n-Butyl, Isobutyl (2-Methylpropyl), sec.-Butyl (1-Methylpropyl), tert.-Butyl (1,1-Dimethylethyl), n-Pentyl oder n-Hexyl. Speziell steht Alkyl für Methyl, Ethyl, n-Propyl, Isopropyl oder Isobutyl.

Im Rahmen der vorliegenden Erfindung steht der Ausdruck geradkettiges oder verzweigtes Alkoxy vorzugsweise für C₁-C₆-Alkoxy und besonders bevorzugt für C₁-C₄-Alkoxy. Alkoxy steht insbesondere für Methoxy, Ethoxy, n-Propyloxy, Isopropyloxy, n-Butyloxy, Isobutyloxy, sec.-Butyloxy, tert.-Butyloxy, n-Pentyloxy oder n-Hexyloxy. Speziell steht Alkoxy für Methoxy, Ethoxy, n-Propyloxy, Isopropyloxy oder Isobutyloxy.

Der Ausdruck "Aryl" umfasst im Rahmen der vorliegenden Erfindung ein- oder mehrkernige aromatische Kohlenwasserstoffreste mit üblicherweise 6 bis 20, vorzugsweise 6 bis 14, besonders bevorzugt 6 bis 10 Kohlenstoffatomen. Beispiele für Aryl sind insbesondere Phenyl, Naphthyl, Indenyl, Fluorenyl, Anthracenyl, Phenanthrenyl, Naphthacenyl, Chrysenyl, Pyrenyl, etc. und speziell Phenyl oder Naphthyl.

Substituierte Aryle können in Abhängigkeit von der Anzahl und Größe ihrer Ringsysteme einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Beispiele für substituierte Arylreste sind 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl, 2,4,6-Tri-tert.-butylphenyl, 1-Methyl-2-naphthyl, 3-Methyl-2-naphthyl, 1,3-Dimethyl-2-naphthyl, 5,6,7,8-Tetramethyl-2-naphthyl, 5-Methyl-2-naphthyl, 6-Methyl-2-naphthyl, 7-Methyl-2-naphthyl, 8-Methyl-2-naphthyl.

Bevorzugt steht R¹ in den Verbindungen der Formel (I) und (II), für unsubstituiertes oder mit 1, 2, 3, 4 oder 5 Substituenten, ausgewählt unter C₁-C₆-Alkyl, und C₁-C₆-Alkoxy, substituiertem Phenyl.

Besonders bevorzugt steht R¹ in den Verbindungen der Formel (I) und (II) für Phenyl, 2-, 3- oder 4-Methylphenyl, 2,4-, 2,5-, 3,5- oder 2,6-Dimethylphenyl, 2,4,6-Trimethylphenyl, 2-, 3- oder 4-Ethylphenyl, 2,4-, 2,5-, 3,5- oder 2,6-Diethylphenyl, 2,4,6-Triethylphenyl, 2-, 3- oder 4-Propylphenyl, 2,4-, 2,5-, 3,5- oder 2,6-Dipropylphenyl, 2,4,6-Tripropylphenyl, 2-, 3- oder 4-Isopropylphenyl, 2,4-, 2,5-, 3,5- oder 2,6-Diisopropylphenyl, 2,4,6-Triisopropylphenyl, 2-, 3- oder 4-Butylphenyl, 2,4-, 2,5-, 3,5- oder 2,6-Dibutylphenyl, 2,4,6-Tributylphenyl, 2-, 3- oder 4-Isobutylphenyl, 2,4-, 2,5-, 3,5- oder 2,6-Diisobutylphenyl, 2,4,6-Triisobutylphenyl, 2-, 3- oder 4-sec-Butyl-phenyl, 2,4-, 2,5-, 3,5- oder 2,6-Di-sec-butylphenyl, 2,4,6-Tri-sec-butylphenyl, 2-, 3- oder 4-tert.-Butylphenyl, 2,4-, 2,5-, 3,5- oder 2,6-Di-tert.-butylphenyl oder 2,4,6-Tri-tert.-butylphenyl.

In einer speziellen Ausführungsform steht R¹ in den Verbindungen der Formel (I) und (II) für Phenyl.

Bevorzugt steht R² in den Verbindungen der Formel (I) und (II) für Wasserstoff und C₁-C₄-Alkyl.

Erfindungsgemäß bevorzugte Bedeutungen für den Rest R² in den Verbindungen der Formel (I) und (II) sind somit beispielsweise Wasserstoff, Methyl, Ethyl, n-Propyl, Isopropyl, n-Butyl, Isobutyl, bevorzugt Methyl, Ethyl, n-Propyl, Isopropyl, n-Butyl und Isobutyl.

In einer speziellen Ausführungsform steht R² in den Verbindungen der Formel (I) und (II) für Methyl.

Bevorzugt steht R³ in den Verbindungen der Formel (I) und (II) für Wasserstoff und C₁-C₄-Alkyl.

Erfindungsgemäß bevorzugte Bedeutungen für den Rest R³ in den Verbindungen der Formel (I) und (II), sind somit beispielsweise Wasserstoff, Methyl, Ethyl, n-Propyl, Isopropyl, n-Butyl, Isobutyl, bevorzugt Methyl, Ethyl, n-Propyl, Isopropyl, n-Butyl und Isobutyl.

In einer speziellen Ausführungsform steht R³ in den Verbindungen der Formel (I) und (II) für Wasserstoff.

Die vorliegende Erfindung betrifft somit im Rahmen einer bevorzugten Ausführungsform ein Verfahren zur Herstellung und Isolierung von 3-Methyl-5-phenyl-pentan-1-ol (Phenoxanol^{®}) der Formel (la)

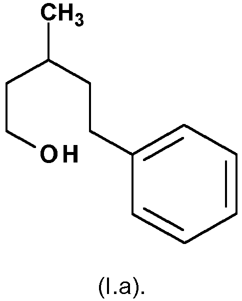

### Schritt a)

Geeignete Ausgangsmaterialien für den Einsatz in Schritt a) können wenigstens eine Verbindung der Formel (II) 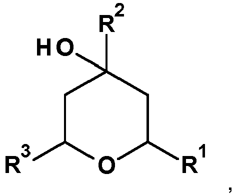 sein, wobei
- R¹: ausgewählt ist unter unsubstituiertem oder mit 1, 2, 3, 4 oder 5 Substituenten, ausgewählt unter C₁-C₁₂-Alkyl, C₁-C₁₂-Alkoxy, Phenyl und Benzyl, substituiertem Aryl mit 6 bis 20 Kohlenstoffatomen;
- R²: ausgewählt ist unter Wasserstoff und C₁-C₆-Alkyl;
- R³: ausgewählt ist unter Wasserstoff und C₁-C₆-Alkyl.

Bevorzugt steht R¹ für unsubstituiertes oder mit 1, 2, 3, 4 oder 5 Substituenten, ausgewählt unter C₁-C₆-Alkyl, und C₁-C₆-Alkoxy, substituierte Phenyl.

Besonders bevorzugt steht R¹ für Phenyl, 2-, 3- oder 4-Methylphenyl, 2,4-, 2,5-, 3,5- oder 2,6-Dimethylphenyl, 2,4,6-Trimethylphenyl, 2-, 3- oder 4-Ethylphenyl, 2,4-, 2,5-, 3,5- oder 2,6-Diethylphenyl, 2,4,6-Triethylphenyl, 2-, 3- oder 4-Propylphenyl, 2,4-, 2,5-, 3,5- oder 2,6-Dipropylphenyl, 2,4,6-Tripropylphenyl, 2-, 3- oder 4-Isopropylphenyl, 2,4-, 2,5-, 3,5- oder 2,6-Diisopropylphenyl, 2,4,6-Triisopropylphenyl, 2-, 3- oder 4-Butylphenyl, 2,4-, 2,5-, 3,5- oder 2,6-Dibutylphenyl, 2,4,6-Tributylphenyl, 2-, 3- oder 4-Isobutylphenyl, 2,4-, 2,5-, 3,5- oder 2,6-Diisobutylphenyl, 2,4,6-Triisobutylphenyl, 2-, 3- oder 4-sec-Butyl-phenyl, 2,4-, 2,5-, 3,5- oder 2,6-Di-sec-butylphenyl, 2,4,6-Tri-sec-butylphenyl, 2-, 3- oder 4-tert.-Butylphenyl, 2,4-, 2,5-, 3,5- oder 2,6-Di-tert.-butylphenyl oder 2,4,6-Tri-tert.-butylphenyl.

In einer speziellen Ausführungsform steht R¹ für Phenyl.

Bevorzugt steht R² für Wasserstoff und C₁-C₄-Alkyl.

Erfindungsgemäß bevorzugte Bedeutungen für den Rest R² in den Verbindungen der Formel (I) und (II), sind somit beispielsweise Wasserstoff, Methyl, Ethyl, n-Propyl, Isopropyl, n-Butyl, Isobutyl, bevorzugt Methyl, Ethyl, n-Propyl, Isopropyl, n-Butyl und Isobutyl.

In einer speziellen Ausführungsform steht R² für Methyl.

Bevorzugt steht R³ für Wasserstoff und C₁-C₄-Alkyl.

Erfindungsgemäß bevorzugte Bedeutungen für den Rest R³ in den Verbindungen der Formel (I) und (II), sind somit beispielsweise Wasserstoff, Methyl, Ethyl, n-Propyl, Isopropyl, n-Butyl, Isobutyl, bevorzugt Methyl, Ethyl, n-Propyl, Isopropyl, n-Butyl und Isobutyl.

In einer speziellen Ausführungsform steht R³ für Wasserstoff.

Der Syntheseweg zur Herstellung der Verbindung der Formel (II) ist in WO 2010/133473, WO 2015/158454 und WO 2014/060345 beschrieben.

### Schritt b)

Erfindungsgemäß wird die Verbindung der Formel (II) einer Eliminierung gefolgt von einer Hydrierung in Gegenwart eines Hydrierkatalysators unter sauren Bedingungen unterzogen. Durch die Eliminierung und Hydrierung im Schritt b) wird die Verbindung der Formel (II) zu der entsprechenden Verbindung der Formel (I) umgewandelt.

Die Eliminierung gefolgt von einer Hydrierung erfolgt vorzugsweise in einer Reaktionsstufe (Eintopfsynthese), d. h. ohne Isolierung von Zwischenverbindungen.

Unter dem Bergiff "unter sauren Bedingungen" wird im Rahmen der Erfindung verstanden, dass die Reaktion in Gegenwart einer Säure stattfindet. Als Säure wird jede Substanz verstanden, die Brönsted-oder Lewis-Acidität aufweist.

Vorzugsweise sind solche Substanzen ausgewählt unter Protonendonatoren, Elektronenakzeptoren und Gemischen davon.

Protonendonatoren sind vorzugsweise ausgewählt unter molekularen Protonensäuren, Ionenaustauschern und Gemischen davon.

Elektronenakzeptoren sind vorzugsweise ausgewählt unter sauren molekularen Elementverbindungen, oxidischen sauren Festkörpern und Gemischen davon.

Geeignete molekulare Protonensäuren sind beispielsweise Salzsäure, Schwefelsäure, Phosphorsäure, Salpetersäure, Essigsäure, Ameisensäure, Trifluormethylsulfonsäure, Methansulfonsäure, p-Toluolsulfonsäure und Gemische davon.

Geigenete saure molekulare Elementverbindungen sind beispielsweise Aluminiumchlorid, Bortrifluorid, Zinkchlorid, Phosphorpentafluorid, Arsentrifluorid, Zinntetrachlorid, Titantetrachlorid, Antimonpentafluorid und Gemische davon.

Geigenete oxidische saure Festkörper sind beispielsweise Zeolithe, Silikate, Aluminate, Alumosilikate, Tone und Gemische davon.

Geigenete Ionenaustauscher sind saure kationische Ionenaustauscher.

Unter dem Begriff "saurer Kationenaustauscher" sind dabei im Rahmen der vorliegenden Erfindung solche Kationenaustauscher in der H⁺-Form zu verstehen, die saure Gruppen, in der Regel Sulfonsäuregruppen, aufweisen, deren Matrix gelförmig bzw. makroporös sein kann. Eine bevorzugte Ausführungsform des erfindungsgemäßen Verfahrens ist dementsprechend dadurch gekennzeichnet, dass man einen sauren, Sulfonsäuregruppen aufweisenden bzw. umfassenden Kationenaustauscher einsetzt.

Saure Kationenaustauscher sind insbesondere lonenaustauscherharze in der H⁺-Form. Als solche kommen beispielsweise in Betracht:
- saure Ionenaustauscher (wie z. B. Amberlyst, Amberlite, Dowex, Lewatit, Purolite, Serdolit), die auf Polystyrol basieren, und die Copolymere aus Styrol und Divinylbenzol als Trägermatrix mit Sulfonsäuregruppen in H⁺-Form enthalten,
- mit Sulfonsäuregruppen (-SO₃H) funktionalisierte lonenaustauschergruppen.

Die Ionenaustauscher unterscheiden sich im Aufbau ihrer Polymergerüste, und man unterscheidet gelförmige und makroporöse Harze. Die sauren lonentauscherharze werden in der Regel mit Salzsäure und/oder Schwefelsäure regeneriert.

Nafion^{®} ist die Bezeichnung der Firma Dupont für perfluorierte polymere lonenaustauscherharze. Es handelt sich dabei um perfluorierte lonenaustauschmaterialien, bestehend aus Fluorkohlenstoff-Basisketten und perfluorierten Seitenketten, die Sulfonsäuregruppen enthalten. Die Harze werden durch eine Copolymerisation von perfluorierten, endständig ungesättigten und mit Sulfonylfluorid funktionalisierten Ethoxylaten mit Perfluorethen hergestellt. Nafion^{®} zählt zu den gelartigen lonenaustauscherharzen. Als Beispiel für ein solches perfluoriertes polymeres lonenaustauscherharz sei Nafion^{®}> NR-50 genannt.

Die sauren Kationenaustauscher werden in der Regel in der H⁺-Form einsetzt, wobei der Ionenaustauscher ein Sulfonsäuregruppen aufweisendes Polymergerüst enthält und entweder gelförmig ist oder makroporöse Harze enthält.

Eine ganz besonders bevorzugte Ausführungsform des erfindungsgemäßen Verfahrens ist dadurch gekennzeichnet, dass der Ionenaustauscher auf einem Polystyrolgerüst mit Sulfonsäuregruppen oder auf einem perfluorierten lonenaustauscherharz mit Sulfonsäuregruppen basiert.

Die kommerziell verfügbaren sauren Kationenaustauscher sind unter den Handelsnamen Lewatit^{®} (Lanxess), Purolite^{®} (The Purolite Company), Dowex^{®} (Dow Chemical Company), Amberlite^{®} (Rohm and Haas Company), Amberlyst^{™} (Rohm and Haas Company) bekannt. Als erfindungsgemäß bevorzugte saure Kationenaustauscher seien beispielsweise genannt: Lewatit^{®} K 1221, Lewatit^{®} K 1461, Lewatit^{®} K 2431, Lewatit^{®} K 2620, Lewatit^{®} K 2621, Lewatit^{®} K 2629, Lewatit^{®} K 2649, Amberlite^{®} IR 120, Amberlyst^{®}131, Amberlyst^{®}15, Amberlyst^{®} 31, Amberlyst^{®} 35, Amberlyst^{®} 36, Amberlyst^{®} 39, Amberlyst^{®} 46, Amberlyst^{®} 70, Purolite^{®} SGC650, Purolite^{®} C100H, Purolite^{®} C150H, Dowex^{®} 50X8, Dowex^{®} 88, Serdolit^{®} rot und Nation^{®} NR-50.

Im Rahmen einer bevorzugten Ausführungsform führt man die erfindungsgemäß durchzuführende Umsetzung von Verbindung (II) in Gegenwart mindestens eines sauren Kationenaustauschers durch, der ausgewählt ist aus der Gruppe der Kationenaustauscher umfassend Lewatit^{®} K 1221, Lewatit^{®} K 2629, Amberlyst^{®} 131, Amberlyst^{®} 35 Purolite^{®} SGC650, Purolite^{®} C100H, Purolite^{®} C15OH, Amberlite^{®} IR 120, Dowex^{®} 88, und Dowex^{®} 50X8.

Erfindungsgemäß insbesondere bevorzugte saure Kationentauscher sind die Kationenaustauscher Amberlyst^{®} 35, Dowex^{®} 88, und/oder Amberlite^{®} IR 120.

Ein erfindungsgemäß ganz besonders bevorzugter saurer Kationenaustauscher ist Amberlyst^{®} 35, der wie die anderen genannten Kationenaustauscher kommerziell verfügbar ist.

Die sauren lonentauscherharze werden in der Regel mit Salzsäure und/oder Schwefelsäure regeneriert.

Die Hydrierung im Schritt b) kann auf an sich herkömmliche Weise mit einem Hydrierkatalysator des Stands der Technik ausgeführt werden. Die Hydrierung kann katalytisch entweder in der Gas- oder Flüssigphase erfolgen. Vorzugweise wird die Hydrierung im Schritt b) in flüssiger Phase in Gegenwart eines heterogenen Hydrierkatalysators und eines wasserstoffhaltigen Gases durchgeführt.

Als Hydrierkatalysatoren kommen prinzipiell alle zur Hydrierung von ungesättigten organischen Verbindungen geeigneten homogenen und heterogenen Katalysatoren in Betracht. Dazu zählen z. B. Metalle, Metalloxide, davon verschiedene Metallverbindungen oder Gemische davon. Geeignete Hydrierkatalysatoren enthalten vorzugsweise wenigstens ein Übergangsmetall, bevorzugt aus den Nebengruppen I. und VI. bis VIII. des Periodensystems der Elemente. Dazu zählen vorzugsweise Pd, Pt, Ni, Rh, Ru, Co, Fe, Zn, Cu, Re oder deren Mischungen.

Der Hydrierkatalysator kann wenigstens ein weiteres Metall/Element enthalten, das von den zuvor beschriebenen Metallen verschieden ist. Das weitere Metall/Element ist vorzugsweise ausgewählt unter Alkalimetallen, Erdalkalimetalle, Aluminium, Silizium, Lanthanoide und Mischungen davon.

Der Anteil des weiteren Metalls/Element ist vorzugsweise im Bereich von 0,1 bis 10 Gew.-% bezogen auf das Gesamtgewicht des aktiven Teil des Hydrierkatalysators (exklusiv Träger).

Die Katalysatoren können allein aus den Aktivkomponenten bestehen, oder die Aktivkomponenten können auf Trägern aufgebracht sein. Als Trägermaterialien eignen sich z. B. Zirkoniumdioxid, Bariumoxid, Zinkoxid, Magnesiumoxid, Titanoxid, Aluminumoxid, TiO₂-Al₂O₃, ZrO₂-Al₂O₃, Zeolithen, Hydrotalcit, Siliciumcarbid, Wolframcarbid, Siliciumdioxid, Kohlenstoff, speziell Aktivkohle oder sulfatierter Kohlenstoff, Diatomeenerde, Tonerde, Bariumsulfat, Calciumcarbonat und Mischungen.

In einer Ausführungsform umfassen die Trägermaterialen gleichzeitig eine erfindungsgemäß eingesetzte Säure oder bestehen daraus.

Zur Erhöhung der katalytischen Aktivität können Ni, Cu oder Co, auch in Form der Raney-Katalysatoren, Pd, Pt, Rh, Ru, Co, Fe, Zn, Cu, oder deren Mischungen als Metallschwamm mit einer sehr großen Oberfläche verwendet werden.

Bevorzugt wird für die Hydrierung im Schritt b) des erfindungsgemäßen Verfahrens vorteilhaft Palladium auf Kohlenstoff, Palladium auf Al₂O₃, Palladium auf SiO₂ oder Platin auf Kohlenstoff eingesetzt. Besonders bevorzugt wird vorteilhaft Palladium auf Kohlenstoff eingesetzt.

Andere geeignete Katalysatoren enthalten z. B. 80 bis 100 Gew.-% Nickel und/oder Cobalt und bis zu 20 Gew.-% aktivierende Metalle wie Kupfer und/oder Chrom. Besonders vorteilhaft werden solche Katalysatoren als Trägerkatalysatoren verwendet.

Der Gehalt an katalytisch aktiven Metallen solcher Trägerkatalysatoren, wobei das Trägermaterial Kohlenstoff ist, beträgt in der Regel 0,05 bis 10 Gew.-%, bezogen auf die Summe von katalytisch aktiven Metallen und Träger.

Der Gehalt an katalytisch aktiven Metallen solcher Trägerkatalysatoren, wobei das Trägermaterial ein Oxid, z. B. Al₂O₃ oder SiO₂ ist, beträgt in der Regel 0,01 bis 1 Gew.- %, bezogen auf die Summe von katalytisch aktiven Metallen und Träger.

Die Katalysatoren können zur Hydrierung in Schritt b) als Formkörper eingesetzt werden. Beispiele umfassen Katalysatorextrudate, wie Stränge, Rippstränge und andere Extrudatformen, Schalenkatalysatoren, Tabletten, Ringe, Kugeln, Splitt, etc.

Bevorzugt wird die Hydrierung in Schritt b) bei einer Temperatur von 60 bis 200 °C, vorzugsweise 120 bis 150 °C durchgeführt.

Sofern man die Umsetzung in der Gasphase durchführt, liegt der Druck vorzugsweise in einem Bereich von 0,9 bis 50 bar, besonders bevorzugt 1 bis 20 bar.

Sofern man die Umsetzung in der Flüssigphase durchführt, liegt der Druck vorzugsweise in einem Bereich von 0,9 bis 200 bar, insbesondere von 40 bis 80 bar.

Die Hydrierung in Schritt b) kann in einem Reaktor oder mehreren hintereinander geschalteten Reaktoren durchgeführt werden. Die Hydrierung kann diskontinuierlich oder kontinuierlich erfolgen. Zur diskontinuierlichen Hydrierung kann z. B. ein Druckgefäß eingesetzt werden. Geeignete Druckgefäße sind z. B. Autoklaven, die mit einer Vorrichtung zum Beheizen und zum Rühren des Reaktorinhalts ausgestattet sind. Bevorzugt erfolgt die Hydrierung in der Flüssigphase über einem Festbett, vorzugsweise in Sumpf- oder Rieselfahrweise oder in Form einer Suspensionskatalyse. Dabei werden die Katalysatoren vorzugsweise als Formkörper eingesetzt, z. B. in Form von gepressten Zylindern, Tabletten, Pastillen, Wagenrädern, Ringen, Sternen oder Strangpresslingen, wie Vollsträngen, polylobären Strängen, Hohlsträngen, Wabenkörpern, etc.

Bei der Suspensionsfahrweise werden ebenfalls heterogene Katalysatoren eingesetzt. Die heterogenen Katalysatoren werden dabei zumeist in feinverteiltem Zustand eingesetzt und liegen im Reaktionsmedium feinteilig suspendiert vor.

Bei der Hydrierung an einem Festbett wird ein Reaktor eingesetzt, in dessen Innenraum ein Festbett angeordnet ist, durch das das Reaktionsmedium strömt. Das Festbett kann dabei aus einer einzigen oder aus mehreren Schüttungen gebildet sein. Jede Schüttung kann dabei eine oder mehrere Zonen aufweisen, wobei wenigstens eine der Zonen ein als Hydrierkatalysator aktives Material enthält. Jede Zone kann dabei ein oder mehrere verschiedene katalytisch aktive Materialien aufweisen und/oder ein oder mehrere verschiedene inerte Materialien aufweisen. Verschiedene Zonen können jeweils gleiche oder verschiedene Zusammensetzungen aufweisen. Es ist auch möglich, mehrere katalytisch aktive Zonen vorzusehen, die beispielsweise durch inerte Schüttungen voneinander getrennt sind. Die einzelnen Zonen können auch unterschiedliche katalytische Aktivität aufweisen. Dazu können verschiedene katalytisch aktive Materialien eingesetzt werden und/oder wenigstens einer der Zonen ein inertes Material beigemischt werden. Das Reaktionsmedium, das durch das Festbett strömt, enthält erfindungsgemäß mindestens eine flüssige Phase. Das Reaktionsmedium kann auch zusätzlich eine gasförmige Phase enthalten.

Als Reaktoren bei der Hydrierung in Suspension kommen insbesondere Schlaufenapparate, wie Strahlschlaufen oder Propellerschlaufen, Rührkessel, die auch als Rührkesselkaskaden ausgestaltet sein können, Blasensäulen oder Air-Lift-Reaktoren zum Einsatz.

Vorzugsweise erfolgt die Hydrierung in Schritt b) in der Suspensionsfahrweise.

Die Hydrierung kann ohne oder mit Zusatz eines Lösungsmittels erfolgen. Als Lösungsmittel kommen Alkohole, Ether, Kohlenwasserstoffe, wie beispielsweise Methanol, Ethanol, Isopropanol, Dioxan, Tetrahydrofuran, n-Pentan, Hexan, Cyclohexan, Toluol, etc., in Frage.

In einer Ausführungsform erfolgt die Hydrierung in Schritt b) ohne Zusatz eines Lösungsmittels.

In einer anderen Ausführungsform erfolgt die Hydrierung in Schritt b) mit Zusatz eines Lösungsmittels.

Zur Hydrierung in Schritt b) kann man die in Schritt a) erhaltene Verbindung der Formel (II) mit einem wasserstoffhaltigen Gas und einem Hydrierkatalysator in Kontakt bringen. Geeignete wasserstoffhaltige Gase sind ausgewählt unter Wasserstoff und Gemischen von Wasserstoff mit wenigstens einem inerten Gas. Geeignete inerte Gase sind z. B. Stickstoff oder Argon. Bevorzugt wird zur Hydrierung in Schritt b) Wasserstoff in unverdünnter Form, üblicherweise in einer Reinheit von etwa 99,9 Vol.-%, eingesetzt.

Durch die Hydrierung in Schritt b) werden die Verbindungen der Formel (II) in 5-Aryl-pentanole (I) überführt. Bevorzugt enthält das zur Hydrierung eingesetzte Ausgangsmaterial Verbindungen der Formel (II), wobei der Reste R¹, R² und R³ die oben genannten Bedeutungen und Bevorzugungen aufweisen. In einer bevorzugten Ausführungsform steht R¹ bevorzugt für Phenyl, R² für Methyl und R³ für Wasserstoff.

In einer speziellen Ausführungsform werden durch die Hydrierung in Schritt b) die Verbindungen (II) in Verbindung der Formel (la), 3-Methyl-5-phenyl-pentan-1-ol überführt.

Die in Schritt b) erhaltene Verbindung der Formel (I) lässt sich durch einfache Reinigungsschritte in eine zur kommerziellen Verwendung geeignete Form überführen.

Falls gewünscht, kann die in Schritt b) erhaltene Verbindung der Formel (I) einer weiteren Aufarbeitung unterzogen werden. Dazu kann die in Schritt b) erhaltene Verbindung (I) prinzipiell üblichen, dem Fachmann bekannten Reinigungsverfahren unterzogen werden. Dazu zählt beispielsweise eine Filtration, eine Neutralisation, eine Destillation, eine Extraktion oder eine Kombination davon.

Bevorzugt wird aus dem in Schritt b) erhaltenen Hydrierprodukt eine an 5-Aryl-pentalolen (I) angereicherte Fraktion und eine an 5-Aryl-pentalolen (I) abgereicherte Fraktion isoliert.

Bevorzugt wird die in Schritt b) erhaltene Verbindung (I) einer destillativen Auftrennung unterzogen. Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc. und Kombinationen davon.

Bevorzugt wird die in Schritt b) erhaltene Verbindung (I) in Schritt c) einer destillativen Auftrennung in wenigstens einer Destillationskolonne unterzogen, die mit trennwirksamen Einbauten versehen ist.

Zur Entfernung von weiteren wasserlöslichen Verunreinigungen kann die in Schritt c) erhaltene an 5-Aryl-pentalolen (I) angereicherte Fraktion wenigstens einem Waschschritt mit Wasser unterzogen werden. Alternativ oder zusätzlich kann die in Schritt c) erhaltene an 5-Aryl-pentalolen (I) angereicherte Fraktion einer weiteren destillativen Aufreinigung unterzogen werden.

Die folgenden Beispiele dienen der Verdeutlichung der Erfindung, ohne sie in irgendeiner Weise zu beschränken.

### BEISPIELE

Gaschromatographische Analysen wurden nach der folgenden Methode durchgeführt:
Säule: ZB5 30 m x 0,25 mm;
FD 1 µm;
Injektortemperatur: 200 °C; Detektortemperatur 280 °C;

| | |
|---|---|
| Temperaturprogramm: | Anfangstemp.: 40 °C, 5 min halten, mit 10 °C/min auf 300 °C, 14 Min isotherm; |
| Retentionszeiten: | 2-Phenyl-4-Hydroxy-4-methyl-tetrahydropyran (Isomere) 27,41 min; 27,68 min |
| | 3-Methyl-5-phenylpentanol: 26,41 min |

Konzentrationen der erhaltenen Rohprodukte (Gew.-%) wurden GC-analytisch mit internem Standard ermittelt.

### Herstellung von 3-Methyl-5-phenylpentan-1-ol ausgehend von 2-Phenyl-4-hydroxy-4-methyl-tetrahydropyran in Methanol

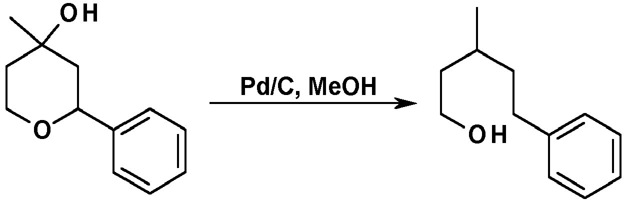

### Beispiel 1:

6 g 2-Phenyl-4-Hydroxy-4-methyl-tetrahydropyran, 60 g Methanol, 0,2 g Katalysator (5 % Pd auf Kohle) und 0,2 g des sauren lonentauschers Amberlyst 35 werden in einen Autoklaven eingewogen. Dieser wird verschlossen und in die Autoklavenstation eingebaut. Anschließend wird der Autoklav einmal mit Stickstoff und einmal mit Wasserstoff gespült. Anschließend werden 30 bar Wasserstoff aufgepresst. Nach Aufheizen auf 120 °C wird auf 50 bar mit Wasserstoff nachgepresst. Die Reaktions-mischung wird 6 Stunden bei diesen Bedingungen gerührt. Bei Vollumsatz des Eduktes finden sich 65,8 % Produkt.

### Beispiel 2:

6 g 2-Phenyl-4-Hydroxy-4-methyl-tetrahydropyran, 20 g Methanol, 0,1 g Katalysator (10 % Pd auf Kohle) und 0,2 g des sauren lonentauschers Amberlyst 35 werden in einen Autoklaven eingewogen. Dieser wird verschlossen und in die Autoklavenstation eingebaut. Anschließend wird der Autoklav einmal mit Stickstoff und einmal mit Wasserstoff gespült. Anschließend werden 30 bar Wasserstoff aufgepresst. Nach Aufheizen auf 140 °C wird auf 50 bar mit Wasserstoff nachgepresst. Die Reaktions-mischung wird 6 Stunden bei diesen Bedingungen gerührt. Bei Vollumsatz des Eduktes finden sich mit 31 % Produkt.

## Patentansprüche

1. Verfahren zur Herstellung von Verbindungen der allgemeinen Formel (I) 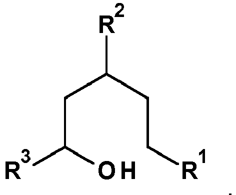 wobei
R¹ ausgewählt ist unter unsubstituiertem oder mit 1, 2, 3, 4 oder 5 Substituenten, ausgewählt unter C₁-C₁₂-Alkyl, C₁-C₁₂-Alkoxy, Phenyl und Benzyl, substituiertem Aryl mit 6 bis 20 Kohlenstoffatomen;
R² ausgewählt ist unter Wasserstoff und C₁-C₆-Alkyl;
R³ ausgewählt ist unter Wasserstoff und C₁-C₆-Alkyl;
umfassend die Schritte:
a) Bereitstellung wenigstens einer Verbindung der allgemeinen Formel (II) 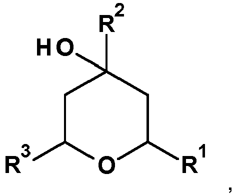 wobei R¹, R² und R³ die zuvor angegebenen Bedeutungen haben,
b) Hydrierung der Verbindung (II) in Gegenwart eines Hydrierkatalysators unter sauren Bedingungen.

2. Verfahren nach Anspruch 1, wobei R¹ unsubstituiertes oder mit 1, 2, 3, 4 oder 5 Substituenten, ausgewählt unter C₁-C₆-Alkyl, C₁-C₆-Alkoxy, substituiertem Phenyl ist.

3. Verfahren nach einem der vorhergehenden Ansprüche, wobei R¹ für Phenyl, 2-, 3- oder 4-Methylphenyl, 2,4-, 2,5-, 3,5- oder 2,6-Dimethylphenyl, 2,4,6-Trimethylphenyl, 2-, 3- oder 4-Ethylphenyl, 2,4-, 2,5-, 3,5- oder 2,6-Diethylphenyl, 2,4,6-Triethylphenyl, 2-, 3- oder 4-Propylphenyl, 2,4-, 2,5-, 3,5- oder 2,6-Dipropylphenyl, 2,4,6-Tripropylphenyl, 2-, 3- oder 4-Isopropylphenyl, 2,4-, 2,5-, 3,5- oder 2,6-Diisopropylphenyl, 2,4,6-Triisopropylphenyl, 2-, 3- oder 4-Butylphenyl, 2,4-, 2,5-, 3,5- oder 2,6-Dibutylphenyl, 2,4,6-Tributylphenyl, 2-, 3- oder 4-Isobutylphenyl, 2,4-, 2,5-, 3,5- oder 2,6-Diisobutylphenyl, 2,4,6-Triisobutylphenyl, 2-, 3- oder 4-sec-Butyl-phenyl, 2,4-, 2,5-, 3,5- oder 2,6-Di-sec-butylphenyl, 2,4,6-Tri-sec-butylphenyl, 2-, 3- oder 4-tert.-Butylphenyl, 2,4-, 2,5-, 3,5- oder 2,6-Di-tert.-butylphenyl oder 2,4,6-Tri-tert.-butylphenyl, bevorzugt für Phenyl steht.

4. Verfahren nach einem der vorhergehenden Ansprüche, wobei R² ausgewählt ist unter Wasserstoff und C₁-C₄-Alkyl, bevorzugt Methyl ist.

5. Verfahren nach einem der vorhergehenden Ansprüche, wobei R³ ausgewählt ist unter Wasserstoff und C₁-C₄-Alkyl, bevorzugt Wasserstoff ist.

6. Verfahren nach einem der vorhergehenden Ansprüche, wobei die Hydrierung in Schritt b) in Gegenwart einer Säure, ausgewählt unter wenigstens einer Protonensäure, wenigstens einer Lewissäure, wenigstens einem sauren Ionenaustauscher, wenigstens einem oxidischen sauren Festkörper, wenigstens einer sauren molekularen Elementverbindung und Gemischen davon, stattfindet.

7. Verfahren nach einem der Ansprüche 1 bis 6, wobei die Hydrierung in Schritt b) in Gegenwart einer Säure erfolgt, die ausgewählt ist unter Salzsäure, Schwefelsäure, Phosphorsäure, Salpetersäure, Essigsäure, Ameisensäure, Trifluormethylsulfonsäure, Methansulfonsäure, p-Toluolsulfonsäure, Aluminiumchlorid, Bortrifluorid, Zinkchlorid, Phosphorpentafluorid, Arsentrifluorid, Zinntetrachlorid, Titantetrachlorid, Antimonpentafluorid und Gemischen davon.

8. Verfahren nach einem der Ansprüche 1 bis 6, wobei die Hydrierung in Schritt b) in Gegenwart eines sauren Kationenaustauschers durchgeführt wird.

9. Verfahren nach einem der Ansprüche 1 bis 6, wobei die Hydrierung in Schritt b) in Gegenwart eines oxidischen sauren Festkörper durchgeführt wird, der ausgewählt ist unter Zeolithen, Silikaten, Aluminaten, Alumosilikaten und Tonen.

10. Verfahren nach einem der vorhergehenden Ansprüche, wobei der Katalysator wenigstens ein Übergangsmetall umfasst, das ausgewählt ist unter Pd, Pt, Ni, Rh, Ru, Co, Fe, Zn, Cu, Re oder Gemischen davon.

11. Verfahren nach einem der vorhergehenden Ansprüche, wobei es sich bei dem Hydrierungskatalysator um einen geträgerten Katalysator handelt.

12. Verfahren nach einem der vorhergehenden Ansprüche, wobei der Träger des Katalysators ausgewählt ist unter Zirkoniumdioxid, Zinkoxid, Magnesiumoxid, Titanoxid, Aluminumoxid, Bariumoxid, TiO₂-Al₂O₃, ZrO₂-Al₂O₃, Zeolithen, Hydrotalcit, Siliciumcarbid, Wolframcarbid, Siliciumdioxid, Kohlenstoff, speziell Aktivkohle oder sulfatiertem Kohlenstoff, Diatomeenerde, Tonerde, Bariumsulfat, Calciumcarbonat und Mischungen davon.

13. Verfahren nach einem der vorhergehenden Ansprüche, wobei die Temperatur in Schritt b) im Bereich von 60 bis 200 °C, bevorzugt im Bereich von 120 bis 150 °C, liegt.

14. Verfahren nach einem der vorhergehenden Ansprüche, wobei der Druck in Schritt b) in einem Bereich von 900 mbar bis 200 bar, bevorzugt 40 bis 80 bar, liegt.

15. Verfahren nach einem der vorhergehenden Ansprüche, wobei es sich um eine Eintopfsynthese handelt.

## Claims

1. A method for preparing compounds of the general formula (I) 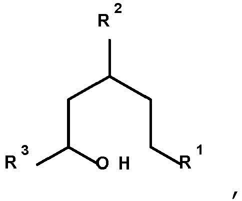 wherein
R¹ is selected from aryl having 6 to 20 carbon atoms that is unsubstituted or substituted by 1, 2, 3, 4 or 5 substituents selected from C₁-C₁₂-alkyl, C₁-C₁₂-alkoxy, phenyl and benzyl;
R² is selected from hydrogen and C₁-C₆-alkyl;
R³ is selected from hydrogen and C₁-C₆-alkyl;
comprising the steps of:
a) providing at least one compound of the general formula (II) 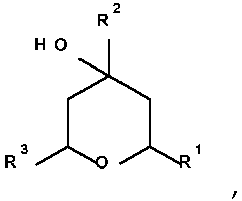 where R¹, R² and R³ have the definitions specified above,
b) hydrogenation of the compound (II) in the presence of a hydrogenation catalyst under acidic conditions.

2. The method according to claim 1, wherein R¹ is phenyl that is unsubstituted or substituted by 1, 2, 3, 4 or 5 substituents selected from C₁-C₆-alkyl, C₁-C₆-alkoxy.

3. The method according to either of the preceding claims, wherein R¹ is phenyl, 2-, 3- or 4-methylphenyl, 2,4-, 2,5-, 3,5- or 2,6-dimethylphenyl, 2,4,6-trimethylphenyl, 2-, 3- or 4-ethylphenyl, 2,4-, 2,5-, 3,5- or 2,6-diethylphenyl, 2,4,6-triethylphenyl, 2-, 3- or 4-propylphenyl, 2,4-, 2,5-, 3,5- or 2,6-dipropylphenyl, 2,4,6-tripropylphenyl, 2-, 3- or 4-isopropylphenyl, 2,4-, 2,5-, 3,5- or 2,6-diisopropylphenyl, 2,4,6-triisopropylphenyl, 2-, 3- or 4-butylphenyl, 2,4-, 2,5-, 3,5- or 2,6-dibutylphenyl, 2,4,6-tributylphenyl, 2-, 3- or 4-isobutylphenyl, 2,4-, 2,5-, 3,5- or 2,6-diisobutylphenyl, 2,4,6-triisobutylphenyl, 2-, 3- or 4-sec-butylphenyl, 2,4-, 2,5-, 3,5- or 2,6-di-sec-butylphenyl, 2,4,6-tri-sec-butylphenyl, 2-, 3- or 4-tert-butylphenyl, 2,4-, 2,5-, 3,5- or 2,6-di-tert-butylphenyl or 2,4,6-tri-tert-butylphenyl, preferably phenyl.

4. The method according to any of the preceding claims, wherein R² is selected from hydrogen and C₁-C₄-alkyl, preference being given to methyl.

5. The method according to any of the preceding claims, wherein R³ is selected from hydrogen and C₁-C₄-alkyl, preference being given to hydrogen.

6. The method according to any of the preceding claims, wherein the hydrogenation in step b) is carried out in the presence of an acid selected from at least one protic acid, at least one Lewis acid, at least one acidic ion exchanger, at least one oxidic acidic solid, at least one acidic molecular element compound and mixtures thereof.

7. The method according to any of claims 1 to 6, wherein the hydrogenation in step b) is carried out in the presence of an acid which is selected from hydrochloric acid, sulfuric acid, phosphoric acid, nitric acid, acetic acid, formic acid, trifluoromethyl-sulfonic acid, methanesulfonic acid, p-toluenesulfonic acid, aluminum chloride, boron trifluoride, zinc chloride, phosphorus pentafluoride, arsenic trifluoride, tin tetrachloride, titanium tetrachloride, antimony pentafluoride and mixtures thereof.

8. The method according to any of claims 1 to 6, wherein the hydrogenation in step b) is carried out in the presence of an acidic cation exchanger.

9. The method according to any of claims 1 to 6, wherein the hydrogenation in step b) is carried out in the presence of an oxidic acidic solid which is selected from zeolites, silicates, aluminates, aluminosilicates and clays.

10. The method according to any of the preceding claims, wherein the catalyst comprises at least one transition metal selected from Pd, Pt, Ni, Rh, Ru, Co, Fe, Zn, Cu, Re or mixtures thereof.

11. The method according to any of the preceding claims, wherein the hydrogenation catalyst is a supported catalyst.

12. The method according to any of the preceding claims, wherein the catalyst support is selected from zirconium dioxide, zinc oxide, magnesium oxide, titanium oxide, aluminum oxide, barium oxide, TiO₂-Al₂O₃, ZrO₂-Al₂O₃, zeolites, hydrotalcite, silicon carbide, tungsten carbide, silicon dioxide, carbon, especially activated carbon or sulfated carbon, diatomaceous earth, clay, barium sulfate, calcium carbonate and mixtures thereof.

13. The method according to any of the preceding claims, wherein the temperature in step b) is in the range from 60 to 200°C, preferably in the range from 120 to 150°C.

14. The method according to any of the preceding claims, wherein the pressure in step b) is in a range from 900 mbar to 200 bar, preferably 40 to 80 bar.

15. The method according to any of the preceding claims, which is a one-pot synthesis.

## Revendications

1. Procédé de préparation de composés de formule générale (I) 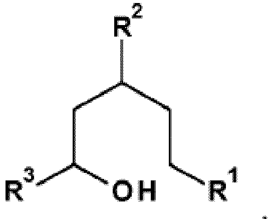
R¹ étant choisi parmi aryle comportant 6 à 20 atomes de carbone non substitué ou substitué par 1, 2, 3, 4 ou 5 substituants choisis parmi C₁₋₁₂-alkyle, C₁₋₁₂-alcoxy, phényle et benzyle ;
R² étant choisi parmi hydrogène et C₁₋₆-alkyle ;
R³ étant choisi parmi hydrogène et C₁₋₆-alkyle ;
comprenant les étapes :
a) mise à disposition d'au moins un composé de formule générale (II) 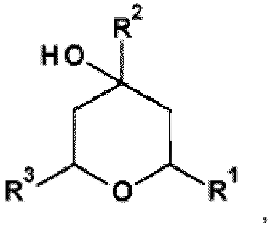 R¹, R², et R³ ayant les significations mentionnées précédemment,
b) hydrogénation du composé (II) en présence d'un catalyseur d'hydrogénation dans des conditions acides.

2. Procédé selon la revendication 1, R¹ étant phényle non substitué ou substitué par 1, 2, 3, 4 ou 5 substituants choisis parmi C₁₋₆-alkyle, C₁₋₆-alcoxy.

3. Procédé selon l'une quelconque des revendications précédentes, R¹ étant phényle, 2-, 3- ou 4-méthylphényle, 2,4-, 2,5-, 3,5- ou 2,6-diméthylphényle, 2,4,6-triméthylphényle, 2-, 3- ou 4-éthylphényle, 2,4-, 2,5-, 3,5- ou 2,6-diéthylphényle, 2,4,6-triéthylphényle, 2-, 3- ou 4-propylphényle, 2,4-, 2,5-, 3,5- ou 2,6-dipropylphényle, 2,4,6-tripropylphényle, 2-, 3- ou 4-isopropylphényle, 2,4-, 2,5-, 3,5- ou 2,6-diisopropylphényle, 2,4,6-triisopropylphényle, 2-, 3- ou 4-butylphényle, 2,4-, 2,5-, 3,5- ou 2,6-dibutylphényle, 2,4,6-tributylphényle, 2-, 3- ou 4-isobutylphényle, 2,4-, 2,5-, 3,5- ou 2,6-diisobutylphényle, 2,4,6-triisobutylphényle, 2-, 3- ou 4-sec-butylphényle, 2,4-, 2,5-, 3,5- ou 2,6-di-sec-butylphényle, 2,4,6-tri-sec-butylphényle, 2-, 3- ou 4-tert-butylphényle, 2,4-, 2,5-, 3,5- ou 2,6-di-tert-butylphényle ou 2,4,6-tri-tert-butylphényle, préférablement phényle.

4. Procédé selon l'une quelconque des revendications précédentes, R² étant choisi parmi hydrogène et C₁₋₄-alkyle, préférablement méthyle.

5. Procédé selon l'une quelconque des revendications précédentes, R³ étant choisi parmi hydrogène et C₁₋₄-alkyle, préférablement hydrogène.

6. Procédé selon l'une quelconque des revendications précédentes, l'hydrogénation dans l'étape b) ayant lieu en présence d'un acide choisi parmi au moins un acide protonique, au moins un acide de Lewis, au moins un échangeur d'ions acide, au moins un corps solide acide d'oxyde, au moins un composé élémentaire moléculaire acide et des mélanges correspondants.

7. Procédé selon l'une quelconque des revendications 1 à 6, l'hydrogénation dans l'étape b) étant réalisée en présence d'un acide qui est choisi parmi l'acide chlorhydrique, l'acide sulfurique, l'acide phosphorique, l'acide nitrique, l'acide acétique, l'acide formique, l'acide trifluorométhylsulfonique, l'acide méthanesulfonique, l'acide p-toluènesulfonique, le chlorure d'aluminium, le trifluorure de bore, le chlorure de zinc, le pentafluorure de phosphore, le trifluorure d'arsenic, le tétrachlorure d'étain, le tétrachlorure de titane, le pentafluorure d'antimoine et des mélanges correspondants.

8. Procédé selon l'une quelconque des revendications 1 à 6, l'hydrogénation dans l'étape b) étant réalisée en présence d'un échangeur de cations acide.

9. Procédé selon l'une quelconque des revendications 1 à 6, l'hydrogénation dans l'étape b) étant mise en œuvre en présence d'un corps solide acide d'oxyde qui est choisi parmi des zéolithes, des silicates, des aluminates, des aluminosilicates et des argiles.

10. Procédé selon l'une quelconque des revendications précédentes, le catalyseur comprenant au moins un métal de transition qui est choisi parmi Pd, Pt, Ni, Rh, Ru, Co, Fe, Zn, Cu, Re ou des mélanges correspondants.

11. Procédé selon l'une quelconque des revendications précédentes, le catalyseur d'hydrogénation étant un catalyseur supporté.

12. Procédé selon l'une quelconque des revendications précédentes, le support du catalyseur étant choisi parmi le dioxyde de zirconium, l'oxyde de zinc, l'oxyde de magnésium, l'oxyde de titane, l'oxyde d'aluminium, l'oxyde de baryum, TiO₂-Al₂O₃, ZrO₂-Al₂O₃, des zéolithes, l'hydrotalcite, le carbure de silicium, le carbure de tungstène, le dioxyde de silicium, le carbone, notamment le charbon actif ou le carbone sulfaté, la terre à diatomées, l'alumine, le sulfate de baryum, le carbonate de calcium et des mélanges correspondants.

13. Procédé selon l'une quelconque des revendications précédentes, la température dans l'étape b) se situant dans la plage de 60 à 200 °C, préférablement dans la plage de 120 à 150 °C.

14. Procédé selon l'une quelconque des revendications précédentes, la pression dans l'étape b) se situant dans la plage de 900 mbars à 200 bars, préférablement de 40 à 80 bars.

15. Procédé selon l'une quelconque des revendications précédentes, qui est une synthèse en un pot.
